# EUROPEAN PATENT APPLICATION

(11) **EP 3 593 832 A1**
(43) Date of publication of application: **15.01.2020**
(21) Application number: 18182850.0
(22) Date of filing: 11.07.2018
(51) Int. Cl.: A61M 5/142, A61M 5/145, A61M 5/148

(54) **DRUG DELIVERY SYSTEM**

(71) Applicant: Debiotech S.A., 1004 Lausanne (CH)
(72) Inventor: CHAPPEL, Eric, 1004 Lausanne (CH); DUMONT-FILLON, Dimitry, 1004 Lausanne (CH)
(74) Representative: Weihs, Bruno Konrad

(57) **Abstract**

A delivery system configured to be secured on a patient skin which comprises a first container having a first movable wall and a first storage compartment intended to store a medical fluid; a second container having a second movable wall and a second storage compartment; and a skin adherable unit configured to secure the first container and the second container to the patient skin. The first movable wall and the second movable wall are facing each other and define a containers' interface having a cavity. The system further comprises a vent device configured to allow a pressure equilibration between the cavity of the containers' interface and the outside of the delivery system when at least one of the first movable wall and the second movable wall moves.

## Description

### FIELD OF INVENTION

This invention relates to a drug delivery system. In particular, it relates to a drug delivery system comprising a drug container and a pressurized means.

### STATE OF THE ART

Many kinds of delivery device are available such as pen, jet and other device designed to inject a single or multiple dose of drug contained in a cartridge or reservoir. Depending on the application (acute intervention, prevention or long-term treatment) the intended user is a healthcare professional, a caregiver or the patient himself. The main drawback of these devices is that the duration of the injection must be short because the user has to handle the device for the whole duration of the delivery. This means that the solution cannot be viscous, the volume to be injected has to be low, and/or the flow rate must be within a short range.

For example, while pen injectors are preferred to inject aqueous solutions up to 3 mL, on-body-injectors become desirable in case of viscous solutions and/or large volume of drugs. Furthermore, some specific subcutaneous formulations cannot be injected via a pen injector even if they comprise recombinant human hyaluronidase as excipient to lower the resistance of the tissues during the SC injection. As consequence, there is a growing interest in providing an on-body-injector able to infuse various formulations of drugs or solution.

The bolus delivered by an on-body-injector is usually accomplished by moving a plunger inside a cylindrical barrel filled with the drug (the reservoir being prefilled or filled by the user). Various engines are used to move the plunger: preloaded spring, linear motor, stepper motor, induction motor, ... Usually, the on-body-injectors are fully disposable thus such devices shall ideally comprise no complex engine.

Another type of on-body-injectors comprises a flexible pouch (storing the drug) pressurized by an elastomeric bladder filled with a propellant. Such devices comprise several drawbacks:
- The need of an output valve for the filling to prevent free flow,
- The relative large dead volume due to the final collapse of the flexible pouch which is not optimal, notably around the filling port and the outlet port, inducing the presence of air after filling and loss of drug which remains in the soft reservoir after infusion,
- The risk of incomplete infusion if the flexible pouch does not collapse in a homogeneous way, and/or
- The need of an opening in the device to introduced the filled flexible pouch inside the bladder before infusion.

Such structure of device is more adapted to very large volume of reservoir (typ. 100 ml or more) in order to maintain the dead volume less than 10%, preferentially less than 5%, more preferentially less than 2%. Moreover, the mechanical constraints related to the large bladder area that is pressurized lead to the use of a reinforced and heavy housing and thus it cannot be wearable.

For a wearable device directly secured to the patient skin, there is a need of an alternative device structure that:
- optimizes the dead volume,
- limits the manipulation (and therefore the risk of errors),
- provides a compact solution to infuse volumes ranging from 1 to 60 ml (typ. 5 ml),
- provides means to monitor the infusion, and/or
- provides passive means to prevent free flow.

### GENERAL DESCRIPTION OF THE INVENTION

According to a first aspect of the invention, the document describes a delivery system configured to be secured on a patient skin comprising:
- a first container having a first movable wall and a first storage compartment intended to store a medical fluid,
- a second container having a second movable wall and a second storage compartment, and
- a skin adherable unit configured to secure the first container and the second container to the patient skin.
The first movable wall and the second movable wall are facing each other and define a containers' interface having a cavity. The system further comprises a vent device configured to allow a pressure equilibration between the cavity of the containers' interface and the outside of the delivery system when at least one of the first movable wall and the second movable wall moves.

The first movable wall may be configured to change the volume of the first storage compartment. And, the second movable wall may be configured in such a manner that a change of the second storage volume induces a movement of the second movable wall.

At least one of the first storage compartment and the second storage compartment may be initially empty and the volume of the containers' interface cavity may initially be maximal.

At least one of the first container and the second container may comprise a rigid part. Said rigid part may extend along at least one length of at least one of the first container and the second container in at least one dimension.

At least one of the first container and the second container may comprise a protrusion including the vent device. The protrusion may extend toward the opposite container. And/or, the protrusion may extend along at least a part of the peripheral edge of the container interface cavity.

At least a part of the first container and/or the second container may form a part of a housing of the delivery system.

At least one of the first container and the second container may comprise a concave internal structure in which at least one of the first movable wall and the second movable wall move. Preferentially, the concave internal structure may be a rigid structure and may comprise a rigid internal wall of the storage compartment.

The medical fluid may be an incompressible fluid such as a liquid or a compressible fluid (such as a gas).

Preferentially, the first container and the second container are in pressure communication via the first movable wall and the second movable wall. In one embodiment, the first container and the second container may be in pressure communication only when the first movable wall and the second movable wall are at least partially in contact.

The cavity of the containers' interface may comprise a variable volume depending on the movement of at least one of the first movable wall and the second movable wall.

Preferentially, the delivery system comprises a third container configured to store a compressed fluid in a third storage compartment. The delivery system may further comprise a fluid pathway and a fluid communication device configured to allow a fluid communication between the third storage compartment and the second storage compartment when the fluid communication device is in open status. The delivery system may further comprise a triggering device operable coupled with the fluid communication device and configured to change the status of the fluid communication device. The delivery system may further comprise a countdown mechanism configured to activate the triggering device after a predetermined time period.

The delivery system may further comprise a housing including a concave internal structure fitted to at least a part of the external surface of at least one of the first container and the second container.

The delivery system may further comprise a transcutaneous device which is in fluid communication with an outlet of the first storage compartment.

The delivery system may further comprise a valve device which is in fluid communication with an outlet of the first storage compartment.

The delivery system may further comprise a hydrophobic membrane which is in fluid communication with an outlet of the first storage compartment. The hydrophobic membrane may cover the outlet of the first storage compartment.

The delivery system may further comprise a flow restrictor which is in fluid communication with an outlet of the first storage compartment.

The delivery system may further comprise a status indicator which is in fluid communication with an outlet of the first storage compartment.

The delivery system may further comprise a pressure transducer which is in fluid communication with an outlet of the first storage compartment.

According to a second aspect of the invention, the document further describes a delivery system configured to be secured on a patient skin comprising:
- a first container having a first flexible membrane and a first variable storage compartment intended to store a medical fluid,
- a second container having a second variable storage compartment,
- a skin adherable unit configured to secure the first container and the second container to the patient skin, and
- a housing having a first cavity.

The first container is at least partially arranged into the first cavity.

The delivery system further comprises a vent device configured to vent the first cavity and a second flexible membrane having a first side configured to be at least partially in contact with the first flexible membrane in delivery condition. The second variable storage compartment is arranged on another side of second flexible membrane.

### LIST OF FIGURES

The present invention will be better understood at the light of the following detailed description which contains non-limiting examples illustrated by the following figures:
**Figure 1** shows a schematic view of the general concept according to an embodiment.
**Figure 2** shows a schematic view of the general structure according to an embodiment.
**Figures 3a and 3b** show schematic views of the general structure with two distinct arrangements of the second and third containers.
**Figures 4a, 4b****,** **4c and 4d** show schematic views of different housings and/or arrangements.
**Figures 5a, 5b, 5c****,** **5d, 5e, 5f and 5g** show schematic views of different containers' interfaces.
**Figures 6a, 6b, 6c****,** **6d and 6e** show schematic views of different status of the delivery system.
**Figure 7a and 7b** show sectional views of alternative realization modes of a container.
**Figure 8** shows a sectional and exploded view of the first and second containers.
**Figure 9** shows two potential embodiments of the first container.
**Figure 10** shows a potential embodiment of the first or second container.
**Figure 11** shows a potential embodiment of a container.
**Figure 12a, 12b****,** **12c and 12d** show different sectional views of an embodiment.
**Figure 13** shows a sectional and exploded view of a potential embodiment of a container.
**Figure 14** shows a sectional and exploded view of a potential embodiment of a container.
**Figure 15a and 15b** show different views of an embodiment.
**Figure 16a and 16b** show different views of an embodiment.
**Figure 17a, 17b****,** **17c and 17d** show schematic views of different potential arrangements.

### LIST OF ELEMENTS

- 1: Delivery system
- 2: Patient
- 3: Skin-adherable unit
- 4: Valve device / Fluid communication device
- 5: Fluid channel
- 6: Containers' interface
- 6a: First flexible membrane / movable wall
- 6b: Second flexible membrane / movable wall
- 6c: Third flexible membrane / movable wall
- 7: Injection device
- 8: Trigger device
- 9: Indicator
- 10: Housing
- 11: First container
- 11a: Storage compartment of the first container
- 12: Second container
- 12a: Storage compartment of the second container
- 13: Third container
- 13a: Storage compartment of the third container
- 14: Pressurization device/means
- 15: Acting device/means
- 16: Vent device
- 17: Gap
- 18: Outlet port
- 19: Fluid pathway / Flow restrictor
- 20: Rigid part of the first container
- 21: Syringe
- 22: Inlet port
- 23: Concave internal structure of the first container
- 24: Concave internal structure of the second container
- 25: Rigid part of the second container
- 26: Valve device
- 27: Filter
- 28: Flexible sheet
- 29: Movement of collapse
- 30: Cavity
- 31: Channel
- 32: Outlet location
- 33: Length of the container
- 34: Width of the container
- 35: Rigid wall
- 36: First surface
- 37: Second surface
- 38: Proximal part
- 39: Distal part
- 40: Lower face
- 41: Upper face
- 42: View icon
- 43: Junction edge
- 44: Containers' interface cavity
- 45: First cavity
- 46: Second cavity

- 50: Container
- 51: Flexible wall
- 52: Opposite wall

- 100: Delivery system
- 101: First container
- 102: Second container
- 103: Third container
- 104: Indicator device
- 111: First storage compartment
- 112: Second storage compartment
- 113: Third storage compartment
- 121: First flexible membrane
- 122: Second flexible membrane
- 131: First housing
- 132: Second housing
- 140: Cavity
- 141: Vent
- 142: First fluid pathway
- 143: Second fluid pathway
- 145: Trigger device

- 200: Container
- 201: Flexible membrane
- 202: Rigid part of the first container
- 203: Rigid housing
- 204: Vent
- 205: Passage
- 206: Fluid pathway
- 207: Lid
- 208: Concave internal structure
- 209: External surface of the container

- 300: Container
- 301: Flexible membrane
- 302: Rigid part of the container
- 303: Fluid pathway
- 304: Edge of the flexible membrane
- 305: Support
- 306: Vent
- 307: External surface
- 308: Protrusion

- 400: Delivery system
- 401: First container
- 402: Second container
- 403: Third container
- 404: Indicator device
- 411: First storage compartment
- 412: Second storage compartment
- 413: Third storage compartment
- 421: First flexible membrane
- 422: Second flexible membrane
- 431: First rigid shell
- 432: Second rigid shell
- 433: Third rigid shell
- 440: Cavity
- 441: Vent
- 442: First fluid pathway
- 443: Second fluid pathway
- 445: Trigger device
- 446: Outlet
- 447: Inlet
- 451: Rigid part of the first container
- 452: Rigid part of the second container
- 453: Rigid part of the third container

### DETAILED DESCRIPTION OF THE INVENTION

In the following detailed description, reference is made to the accompanying drawings that form a part hereof, and in which are shown by way of illustration several embodiments of devices, systems and methods. It is to be understood that other embodiments are contemplated and may be made without departing from the scope or spirit of the present disclosure. The following detailed description, therefore, is not to be taken in a limiting sense.

All scientific and technical terms used herein have meanings commonly used in the art unless otherwise specified. The definitions provided herein are to facilitate understanding of certain terms used frequently herein and are not meant to limit the scope of the present disclosure.

As used in this specification and the appended claims, the singular forms "a", "an", and "the" encompass embodiments having plural referents, unless the content clearly dictates otherwise.

As used in this specification and the appended claims, any direction referred to herein, such as "top", "bottom", "left", "right", "upper", "lower", and other directions or orientations are described herein for clarity in reference to the figures and are not intended to be limiting of an actual device or system. Devices and systems described herein may be used in a number of directions and orientations.

As used herein, "have", "having", "include", "including", "comprise", "comprising" or the like are used in their open ended sense, and generally mean "including, but not limited to.

As used in this specification and the appended claims, the term "or" is generally employed in its sense including "and/or" unless the content clearly dictates otherwise.

As used in this specification and the appended claims, the expression "at least one of an A and a B" or the like is used in it open ended sense, and generally means "A and/or B".

### 1.1 GENERAL CONCEPT

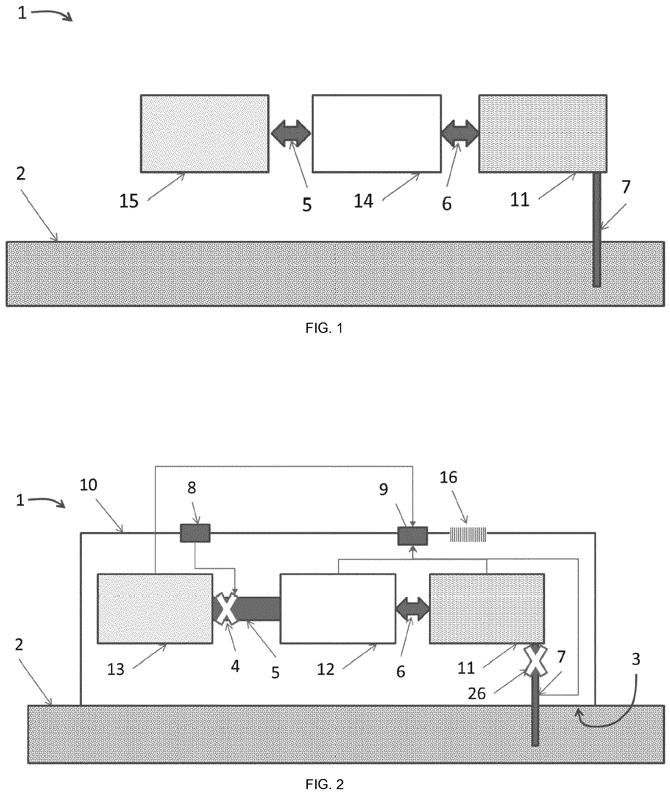

As shown by the figure 1, the delivery system (1) comprises a first container (11) adapted to store a medical fluid (such as a drug or a pharmaceutical fluid or other fluids). In this document, the medical fluid may be also called fluid or solution. The medical fluid may be a liquid or a gas, for example an incompressible fluid, and is intended to be delivered to a patient (2) for example via a transcutaneous device (7). The transcutaneous device (7) may be in fluid communication with the first container (11) and may comprise for example a needle, a cannula, one or more micro needle or other means adapted or configured to bring the medical fluid into or on the patient body.

The delivery system (1) further comprises a pressurization means (14) which may be operatively coupled (6) to the first container (11). For example, the pressurization means (14) may be adapted or configured to pressurize the first container so as to cause the flow of the medical fluid from the first container (11) to the patient (2). For example if the first container (11) comprises a flexible membrane, the pressurization means (14) may be configured to act on the flexible membrane in order to pressurize the first container.

The delivery system (1) may further comprise an action means (15) which may be operatively coupled to the pressurization means (14). For example, the action means (15) may be adapted or configured to allow the pressurization of the first container.

According to a preferred embodiment, the pressurization means (14) comprises a container (for example a second container (12)) and the action means (15) comprises a container (for example a third container (13)) in fluid communication or pressure communication with the pressurization means (14). The action means (15) may further comprise at least one of a valve device and a fluid pathway (5) allowing a fluid communication between the third container and the second container for example when the valve device (4) is in open position. The third container may be configured or adapted to store a compressed fluid such as a propellant. The propellant may be a liquefied gas that exhibits a large value of vapor pressure at ambient temperature. The pressure range is therefore determined by the evolution of this vapor pressure within a predefined range of functioning temperature, typically from 5°C to 40°C. Considering isobutane as propellant, this pressure (absolute) is for instance 1.86 bar at 5°C, 3 bar at 20°C and 5.25 bar at 40°C.

According to another embodiment, the pressurization means (14) may be a spring, an elastic device, an actuator which acts on the first container, a plunger,... And the actions means (15) may comprise an element adapted to generate a gas, such as a battery or other chemical elements, or to act on the pressurization means (14)...

The delivery system may be intended for single use. Thus, after a single use/delivery, the delivery system may be discarded, for example entirely discarded.

The delivery system (1) may be configured to deliver the entire volume of the solution stored in the first storage compartment in a single bolus. Thus, once the trigger device is activated, the delivery system will infuse the solution to the patient until the first storage compartment is substantially empty.

The delivery system may be adapted to inform the patient of the end of the delivery, via an indicator device.

In one embodiment, the delivery system may comprise an electronic device configured to control and/or monitor the delivery. Preferentially, the delivery system does not comprise any electronic element configured to convey the solution and/or to pressurise the first container and/or to control the pressurization means (14) and/or the action means (15). Nevertheless, an indicator device may comprise some electronic elements such as a pressure sensor, a processor and/or a light indicator (LED,...).

In one embodiment, the delivery system may be configured to operate without any electronic element and/or battery.

### 1.2 GENERAL STRUCTURE

The delivery system (1) disclosed by at least one of the figures 2, 3, and 4 comprises at least one of:
- A first container (11)
- A second container (12), and
- A third container (13).

Preferentially, the first container (11) comprises a first storage compartment (11a), a first movable wall (for example a flexible wall/membrane) configured to change a capacity of the first container (11) (for example the volume of the storage compartment (11a)) and an outlet port allowing the solution to get out of the storage compartment (11a). The outlet port is configured to be in fluid communication with the transcutaneous device (7), for example when the delivery system delivers the solution to the patient.

The first container may comprise an inlet port to fill the storage compartment (11a) with the solution intended to be delivery to the patient. The inlet port and the outlet port may be the same port or the inlet port may be a different port from the outlet port.

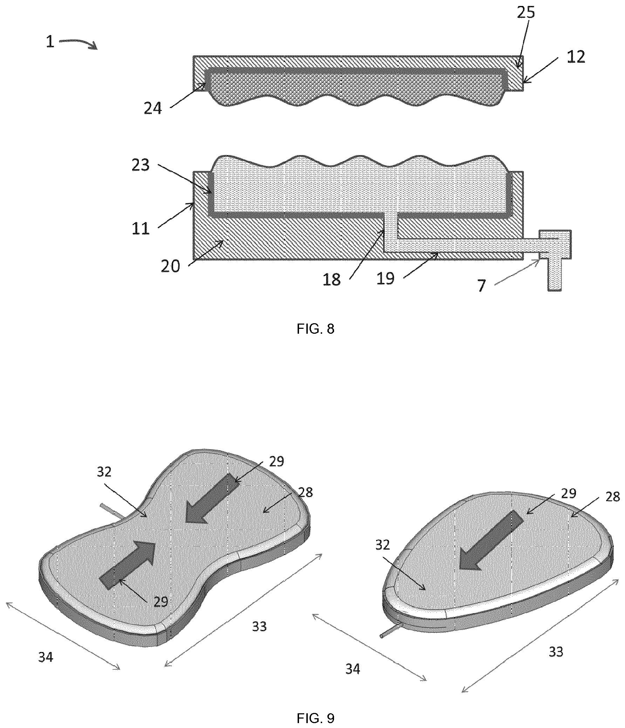

As shown by the figure 8, the delivery system (1) may further comprise a flow restrictor (19) arranged between the outlet port (18) of the first container (11) and the transcutaneous device (7). The flow restrictor (19) may be configured to regulate the flow of solution delivered to the patient when the solution stored in the first container is submitted to a determined range of pressure. For example, the target flow rate may be comprised between 0 and 10 ml/min, preferentially between 0,01 and 5 ml/min and more preferentially between 0.05 and 3 ml/min. The flow restrictor (19) may comprise a fluid pathway configured to provide a predetermined fluidic resistance between the first reservoir and the patient. The flow restrictor (19) may be configured in order to provide the main fluidic resistance from the first storage compartment to an end of the transcutaneous device. The flow restrictor (19) may be arranged into or secured to at least a rigid part (20) of the first container. The flow restrictor (19) may be composed of at least one part.

The fluid pathway is preferentially straight but it may comprise one or several curved sections.

The fluid pathway may comprise a hollow rod which may be made from at least one of the following materials: metal, plastic, silicon, ceramic or glass. The hollow rod may comprise a sharp end (such as a needle) configured to be inserted into the transcutaneous device.

Other examples of flow restrictor (which may be implemented in the delivery system) have been described by at least one of the following documents: WO2010020891 A1, WO2011098946A1, WO2014108860A1 and WO2011098867A1. The contents of these documents are incorporated by reference in the present document.

The delivery system may comprise a valve device (26) arranged between the first storage compartment and the transcutaneous device and configured to control the fluid communication between the first storage compartment and the patient. This valve device may comprise a first position/state/condition in which the valve prevent the flow of the solution to the patient and a second position/state/condition in which the valve allow the solution to flow to the patient. The valve may be a mechanical valve (e.g. mitral valve, duckbill valve...) or a capillary valve (e.g. a hydrophobic membrane...). A hydrophobic membrane may be configured to prevent the flow of liquid as long as the pressure in the liquid is not large enough to overcome this capillary barrier. This later solution may be interesting because the reservoir remains vented after final assembly. This pressure equilibration may be required by the sterilization or the final packaging process.

Preferentially, the second container (12) comprises a second storage compartment (12a), a second movable wall (for example a flexible wall/membrane) configured to change a capacity of the second container (12) (for example the volume of the second storage compartment (12a)). More particularly, a change of the volume of the second storage compartment induces a movement of the second movable wall (for example a movement/deformation/stretching of the flexible membrane). The second container may comprise an inlet port. The second container (12) may comprise an outlet port configured to expel the propellant from the second container for example at the end of the delivery.

Preferentially, the first container (11) and the second container (12) comprise a containers' interface (6) configured in such a manner that the first container and the second container are in pressure communication when the first movable wall and the second movable wall are at least partially in contact.

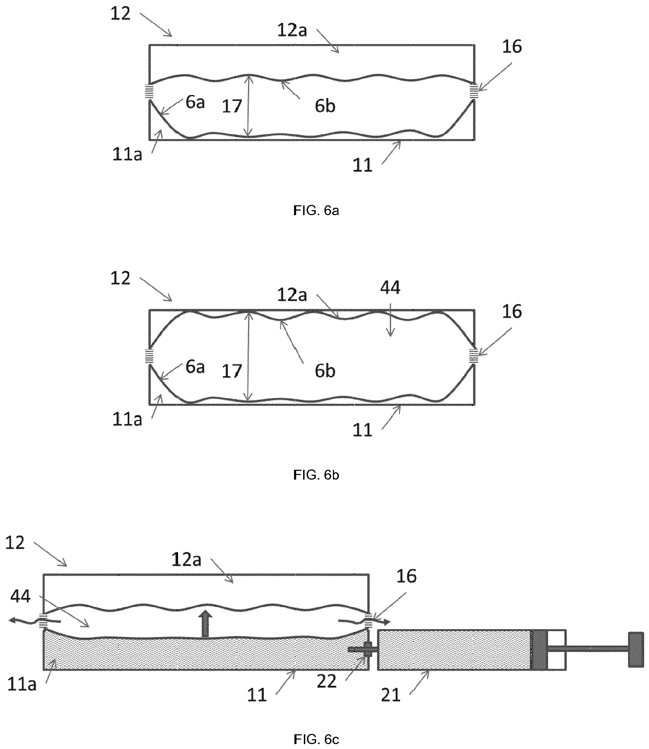

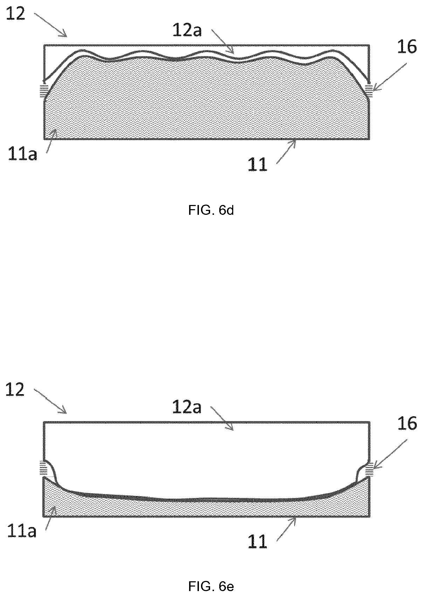

As disclosed by the figures 6, the containers' interface (6) may comprise a cavity defined by at least a first surface (6a) of the first movable wall and a second surface (6b) of the second movable wall. Preferentially, the first surface (6a) is arranged opposite to the second surface (6b). The containers interface (6) may be configured in such manner that the first surface (6a) may be:
- spaced apart from the second surface (6b), when the delivery system (1) is in a first state (for example an initial state of the delivery system), and/or
- in contact with the second surface (6b) when the delivery system (1) is in a second state (for example a delivery state of the delivery system).

The cavity of the containers' interface may have a variable volume depending on the delivery state. This cavity is preferentially vented by a vent device as described below.

The vent device (16) (for example an aperture) is intended to allow a fluid communication between at least an inside part of the delivery system and the outside environment of the delivery system. The vent device may be configured to keep the cavity of the containers' interface (6) vented. In particular, the vent device may be configured to provide a pressure equilibration of the containers' interface cavity when at least one of the first storage compartment volume and the second storage compartment volume varies.

The venting device may comprise a hydrophobic membrane, a filter or a coating configured to prevent the flow of water into the enclosure. The venting device may comprise a baffle configured to prevent the insertion of a straight and rigid tip.

An example of vent device which may be comprised in the delivery system is described by the United States Patent: US9,872,955, the entire disclosure of which is incorporated herein by reference.

Preferentially, the third container (13) comprises a third storage compartment (13a) configured to store a compressed fluid (in a compressed state) such as a propellant. The third container may comprise rigid walls in such a manner the volume of the third storage compartment (13a) is constant. The third container comprises an outlet port configured to be in fluid communication with the second storage compartment in a delivery state in such a manner that the compressed fluid flows from the third storage compartment to the second storage compartment.

The delivery system may comprise a valve device (4) configured to prevent the fluid communication (or to isolate the second storage compartment from the third storage compartment) when the valve device is in a first state/position (for example closed) and to allow the fluid communication between the second storage compartment and the third storage compartment when the valve device in a second state/position (for example open).

The delivery system may comprise a trigger device (8). The trigger device may be configured to initiate a change of delivery system state.

Preferentially the trigger device is operatively coupled to the valve device (4) (for example mechanically). In this case the trigger device may be configured to change the state/position of the valve device (4) in order to pass from a first state (first position) to a second state (second position) and/or vice versa.

The trigger device (8) may comprise a button such as a push or a sliding button, a timer, and/or a countdown mechanism. When the trigger device is activated (for example by the user/patient), the trigger device is configured to initiate the change of the delivery system state or to launch the countdown or the timer.

The delivery system may comprise a status indicator (9) as disclosed by the European patent application number EP18157250.4, the entire disclosure of which is incorporated herein by reference. The indicator device may be operatively coupled to at least one of the first container, the second container, the third container and the transcutaneous device. The indicator device (9) may be configured to provide an information to the user concerning the status of the delivery system or one of the listed elements, for example: Occlusion, ready, ready to be activated, ready for filling, ready to infuse, delivery in progress, error, full storage compartment, empty storage compartment, delivery finished,...

The delivery system may comprise one or several parts. As disclosed by the figure 2, the delivery system may be a single block device. In this case, the delivery system (1) comprises at least one housing (10) in which the first, the second and the third containers are arranged. These three containers may not be separated or removed from the housing (10).

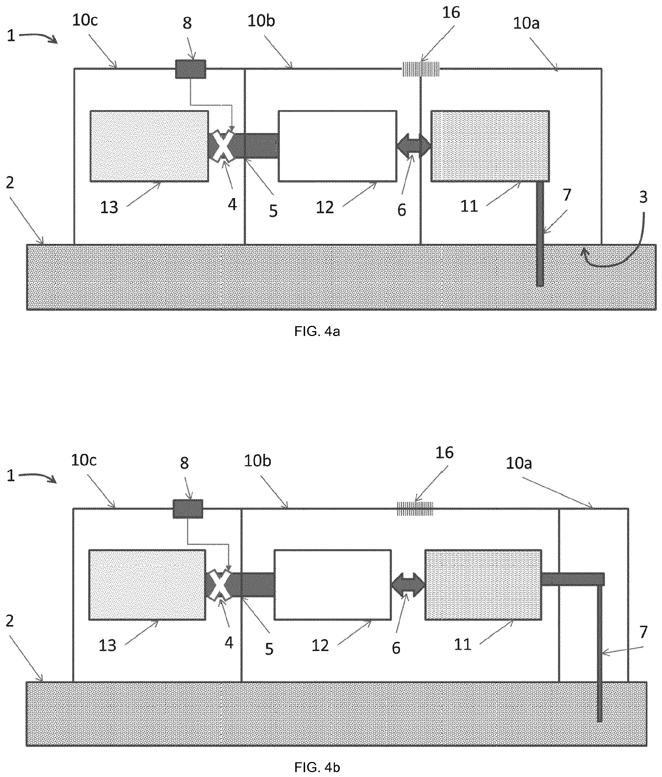

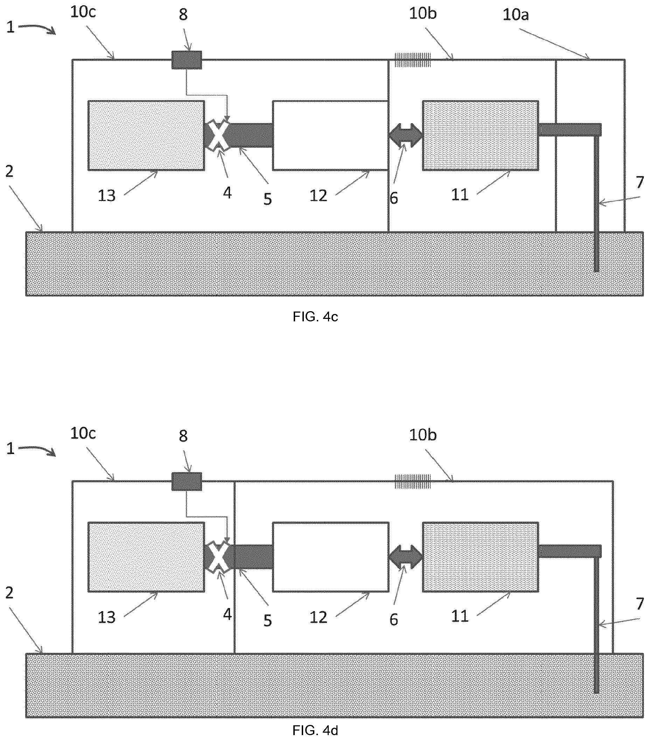

As disclosed by the figures 4a, b and c, the delivery system may be modular and/or may comprise several parts. A first part may comprise a first housing (10a) in which at least one of the first storage container and at least a part of the transcutaneous device may be arranged. A second part may comprise a second housing (10b) in which at least one of the first storage container, the second storage container and at least a part of the transcutaneous device may be arranged. A third part may comprise a third housing (10c) in which at least one of the first storage container, the second storage container, the third storage container and at least a part of the transcutaneous device may be arranged.

The vent device may be arranged on at least one of the first, the second and the third housing, preferentially in fluid communication with the containers' interface (6).

The housings or the containers may be configured to be coupled there between in order to form the delivery system. The coupling process may be performed during the manufacturing process or by a user before the use.

The housing may comprise rectangular shape, trapezoidal shape, egg shape, butterfly shape, disks shape, triangular shape or ellipsoidal shape. Preferentially the housing is thin with smooth angles.

Preferentially, to solve problems related to the recycling of waste, at least one of the second container and the third container may be configured to be removable from the first container, for example at the end of the treatment. Thus at least one of the containers may be discarded in appropriate waste.

At least one of the third container and the second container may be adapted to the compressed fluid (propellant) and/or to the physical features (volume, viscosity,...) of the solution stored in the first container. In other terms, the features (shape, dimension, material, ...) of the third container and/or the second container are designed by taking into account the physical features (volume, viscosity,...) of the compressed fluid (propellant) and/or the physical features (volume, viscosity,...) of the solution stored in the first container.

The delivery system may comprise a skin-adherable unit (3) configured to secure the delivery system to the patient skin. The skin-adherable unit may comprise a first side having an adhesive surface facing toward the skin surface of the patient for adhesion and a removable cover for covering the skin-adherable unit. The skin-adherable unit may further comprise a frame and coupling device configured to (optionally removably) secure the delivery system.

### 1.3 DELIVERY SYSTEM STATES

The figures 6 show different potential states of the delivery system, in particular the first and the second container.

### Initial state:

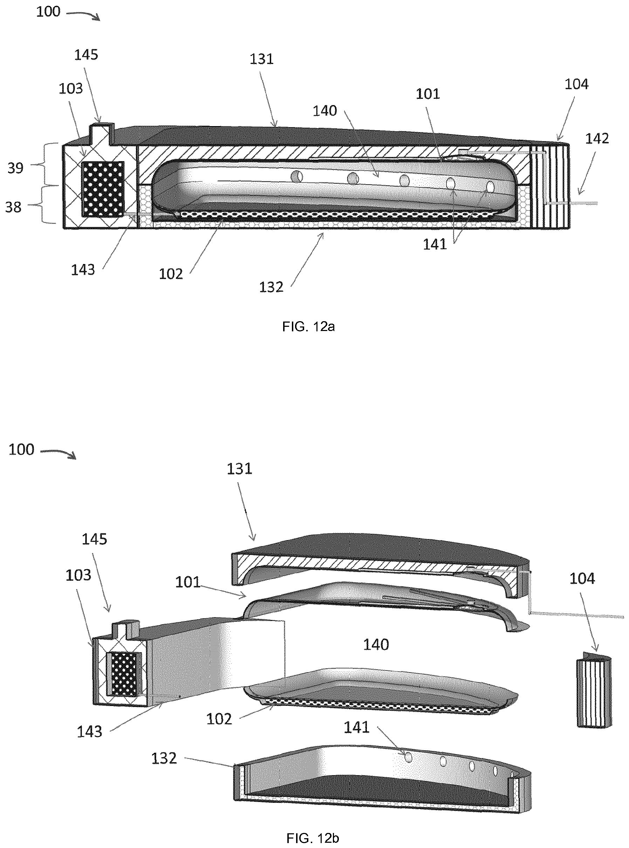

In one embodiment, as disclosed by the figures 6a and b, the first storage compartment does not initially store the solution (for example before the delivery, when the delivery system is in the packaging). Preferentially, the volume of the first storage compartment is initially minimal. In this case, the first storage compartment is initially substantially empty. The movable wall may be in contact (or very close to or in the closest manner) with the opposite internal wall of the first container (for example on a substantial length of the first storage compartment), for example against the internal wall of the rigid part (20) of the first container (11). The figure 12a shows an example of an embodiment in initial sate comprising a first storage compartment which is substantially empty.

In another embodiment (for example as the state of the system shown by the figure 6d), the first storage compartment initially stores the solution. In this case, the volume of the first storage compartment may be maximal.

In both cases, preferentially, the movable walls of the first and the second containers are not in contact in order to not exert any non-intentional pressure to the first container. Furthermore, the containers' interface may be vented by the vent device.

At the initial state of the delivery system, the gap (17) between the first surface (6a) of the first movable wall and a second surface (6b) of the second movable wall may be maximal and/or the volume of the containers' interface cavity (44) may be maximal.

Preferentially, the volume of the second storage compartment is initially minimal. Nevertheless, the second storage compartment may be non-empty and initially store a fluid such as a gas (for example air trapped during the manufacturing process).

As the third container (not shown by the figures 6) preferentially comprises only rigid walls, the volume of the third storage compartment is constant. And, the third storage compartment may or may not initially store the compressed fluid.

### Filling step:

If the first storage compartment does not initially store the solution, the user has to fill the first storage compartment before use and the first container may comprise an inlet port (22) configured to fill the first storage compartment with the solution.

The figure 6c shows the filling of the first container via a syringe (22), the volume of the first storage compartment increases and the volume of the containers' interface cavity decreases. The first surface (6a) of the first movable wall moves toward the second container, for example toward the second surface (6d) of the second movable wall. The fluid (for example the gas trapped into the containers' interface cavity) may be discharged/expelled to outside of the delivery system through the vent device (16).

As disclosed by the figure 8, the second container may comprise a concave internal structure (24) which may be rigid. The second movable wall may be configured to closely match the concave internal structure (24). During the filling step, the first movable wall may move at least partially into the concave internal structure of the second container.

If the third storage compartment does not initially store the compressed fluid, the user has to fill the third storage compartment before use and the third container may comprise an inlet port configured to fill the third storage compartment with the compressed fluid.

### Full drug state:

At the end of the filling step, the gap between the first surface (6a) of the first movable wall and a second surface (6b) of the second movable wall is substantially reduced. The first surface (6a) of the first movable wall and a second surface (6b) of the second movable wall may or may not be in contact. The pressure exerted on the first movable wall, if any, must be limited and not induce a flow of the solution to the patient.

As disclosed by the figure 6d, when the first storage compartment is full, the first movable wall may be at least partially deployed into the concave internal structure (24).

At this state the indicator device may indicate that the delivery system is ready to infuse or the drug container is full.

### Activated / delivery state:

After the trigger device has been activated (for example as soon as or after a predetermined time period), the trigger device initiates a change of the delivery system state. The valve device (4) switches from a first state/position (closed position) to a second state/position (open position). A second state/position of the valve device induces a flow of the compressed fluid from the third storage compartment to the second storage compartment. The second movable wall moves toward the first container due to the fluid pressure of the compressed fluid. The fluid pressure of the compressed fluid into the second container induces a displacement (a bending) of the second movable wall which exerts a pressure on the first movable wall.

At least during the delivery, the second movable wall is configured to come into contact with the first movable wall on at least 50, preferentially at least 80%, more preferentially at least 90% for example (substantially) on 100% of the first movable wall surface.

The fluid pressure of the second container is transmitted (at least partially or substantially) to the first container, in particular to the solution stored in the first storage compartment. Furthermore, the pressure causes a flow of the solution to the transcutaneous device.

The volume of the second storage container and/or the quantity of compressed fluid are adapted in order to have a substantially constant fluid pressure during a predetermined time period of the delivery. For example this predetermined time period may be at least 50% of the time period necessary to substantially drain the first storage compartment, preferentially at least 80%, more preferentially at least 90%, for example at least equal to the time period necessary to substantially drain the first storage compartment. In the latter case, the fluid pressure is constant over the entire duration of the delivery.

### 1.4 CONTAINERS' INTERFACE

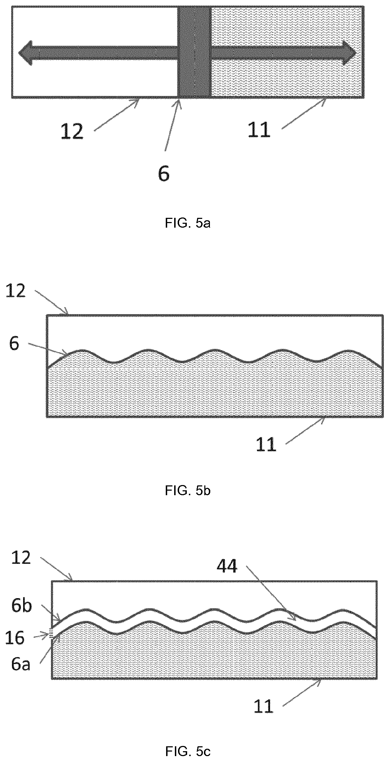

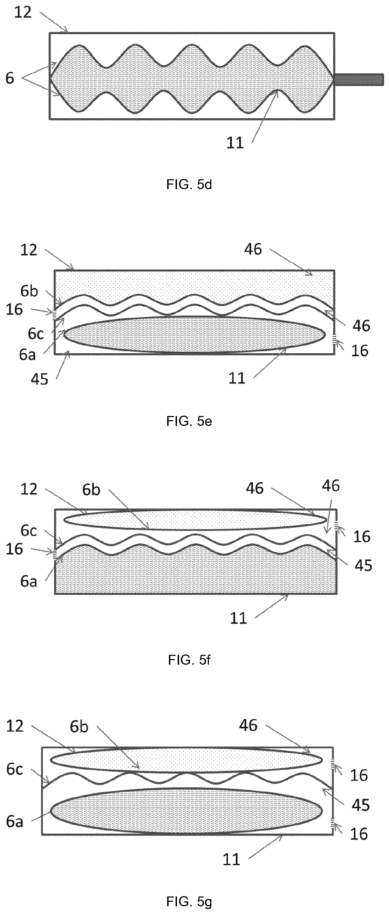

The figures 5a, b, c, d, e, f and g contain non-limiting examples of the containers' interface (6).

In the figure 5a, the containers' interface (6) is at least one plunger delimitating the capacity of the first and the second containers. When the second container (12) is pressurized by a fluid, the plunger is moved causing the flow of solution of the first container (11). In this example, the first and second containers further comprise rigid walls and the plunger slides against the rigid walls.

In the figure 5b, the containers' interface (6) comprises a single flexible membrane wherein the first movable wall and the second movable wall are formed by the single flexible membrane. In this example, the first and/or second containers may further comprise rigid walls. And the single flexible membrane may be configured to move away and/or from the rigid wall of first and/or second containers.

In the figure 5c, the containers' interface (6) comprises two distinct flexible membranes. The first movable wall of the first container (11) comprises a first flexible membrane (6a) . The second movable wall of the second container (12) comprises a second flexible membrane (6b). The first and/or second containers may further comprise rigid walls. The first flexible membrane may be configured to move away from and/or toward the second container (12). The second flexible membrane may be configured to move away from and/or toward the first container (11). The containers' interface (6) may comprise a containers' interface cavity (44). This containers' interface cavity (44) is arranged into the delivery system (for example into a housing). Preferentially, the containers' interface cavity (44) is at least partially defined by the first flexible membrane (6a) and the second flexible membrane (6b). This cavity may be vented by a vent device (16) for pressure equilibration when the volume of at least one of the first container (11), the second container or the enclosure varies or when at least one of the first flexible membrane and the second flexible membrane moves.

In the figure 5d, the containers interface (6) comprises at least one flexible membrane wherein the first container is a flexible container. In this case the first container (11) may be arranged into the second container (12).

According to the figures 5e, 5f and 5g, the containers' interface (6) comprises two distinct flexible membranes. The first movable wall of the first container (11) comprises a first flexible membrane (6a). The second movable wall of the second container (12) comprises a second flexible membrane (6b). The first and/or second containers may further comprise rigid walls or may be a flexible pouch (such as a balloon). The first flexible membrane may be configured to move away from and/or toward the second container (12). The second flexible membrane may be configured to move away from and/or toward the first container (11). The containers' interface (6) may further comprise at least one cavity (45, 46) arranged into the delivery system (for example into a housing).

A first cavity (45) may be defined by at least one of a third flexible membrane (6c) and the first flexible membrane (6a). The third flexible membrane (6c) may be configured to move away from and/or toward the first container (11) (or the second container (12)) and to vary the volume of the first cavity (45). The first cavity may comprise a dedicated vent device (16) configured to vent the first cavity for pressure equilibration for example when the first container (11) is being filled.

A second cavity (46) may be defined by at least one of a third flexible membrane (6c) and the second flexible membrane (6b). The third flexible membrane (6c) configured to move away from and/or toward the first container (11) (or the second container (12)) and to vary the volume of the second cavity (46). The second cavity may comprise a dedicated vent device (16) configured to vent the second cavity for pressure equilibration for example when at least one of the second flexible membrane (6b) and the third flexible membrane (6c) moves or when the volume of at least one of the first storage compartment and the second storage compartment varies.

The third flexible membrane may be configured to provide an additional insulation (shock and/or thermal) to the first container (11). Furthermore, in case of a second container leakage, the fluid initially stored in the second storage compartment may exit by the dedicated vent device (16).

According to the figures 5e and 5g, the first container is arranged into the first cavity (45).

According to the figures 5f and 5g, the second container is arranged into the second cavity (46).

### 1.5 CONTAINERS FEATURES

As disclosed above, the first container (11) may comprise a first movable wall configured to change a capacity of the first container (11) (for example the storage compartment (11a)) and an outlet port configured to be in fluid communication with the transcutaneous device (7). The first movable wall may comprise/be a flexible membrane. The first container may comprise two flexible membranes sealed together. The first container may further comprise a rigid part. The rigid part may comprise at least the outlet port and/or an inlet port configured to fill the storage compartment (11a). The internal walls of the rigid part and of the flexible membrane may define the first storage container.

The storage compartment is configured to receive and store a volume of solution may be comprised between 0 and 100 ml, preferentially between 1 and 10 ml for example 5ml. The viscosity of the solution may be comprised between 0 and 100 cP, preferentially between 1 and 80 cP, more preferentially between 10 and 65 cP at 20°C, for example 25 cP.

One of the inlet port and the outlet port of the first container may be covered by a filter (27) such as a hydrophilic filter configured to allow the passage of liquid and prevent the passage of gas (for example air).

The outlet port may be covered by a hydrophobic filter configured to prevent the flow of the solution outside of the first storage compartment until the pressure of the solution reaches a predetermined threshold. The hydrophobic filter is used here as a valve device in order to infuse the solution only when a predetermined pressure is reached into the first and/or the second storage compartment. The hydrophobic filter is characterized by a threshold pressure that is typically at least 2 times larger than the maximum drug reservoir filling pressure (typically few tenths of mbar due to the reservoir film unfolding) and at least 2 times smaller than the minimum infusion pressure (e.g. the vapour pressure of the propellant at 5°C)

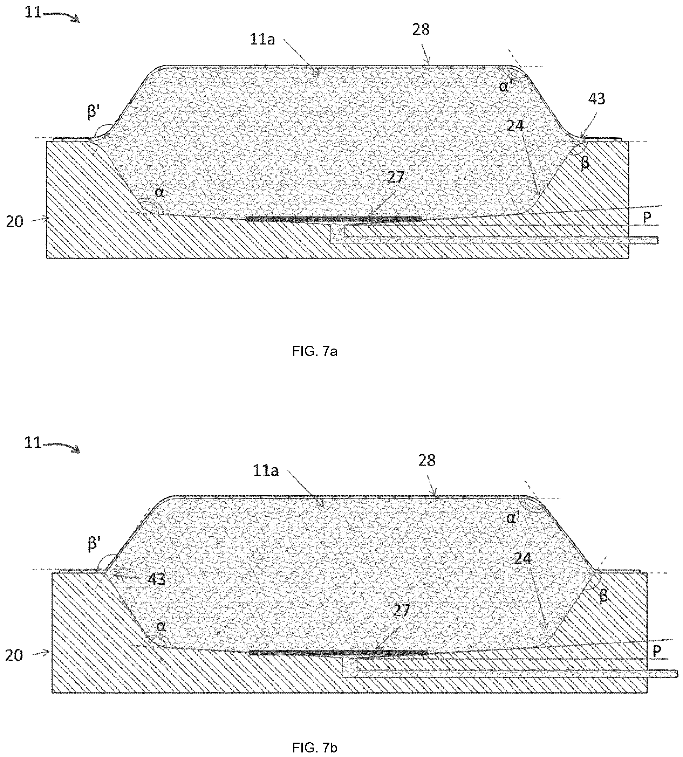

Preferentially, the first container (11) comprises a rigid part (20) and a flexible sheet (28), as described by the figure 7. The flexible sheet may be secured/sealed to the rigid part at a junction edge (for example by thermos, ultrasonic, infrared or laser-welding or gluing). Both define the first storage compartment (11a), in particular, the internal wall of the rigid part and of the flexible sheet. The rigid part (20) may comprise a concave internal structure (24) and the flexible sheet may be configured to come into contact with the internal wall of the rigid part (20), for example, the flexible sheet may be elastic and/or thermoformed.

The concave internal structure (24) is designed in such a manner that the dead volume of the first storage compartment (the residual volume at the end of the delivery) is minimal for example substantially equal to 0. For example, at the end of the treatment, the wall (of the concave internal structure (24)) intended to be in contact with the solution is covered by the flexible sheet and substantially in contact with the flexible sheet. Thus at the end of the treatment (end of delivery), the first container stores a residual volume of solution which is less than 10% of the solution initially stored in the first container, preferentially less than 8%, more preferentially less than 5%. Furthermore, at the end of the treatment (end of delivery), the delivery system comprises a residual volume of solution which is less than 10% of the solution initially stored in the first container, preferentially less than 8%, more preferentially less than 5%.

Furthermore, as described in the figure 7a, the concave internal structure (24) may comprise a first angle β and a second angle o. The first angle β may be rounded with a strictly non-null radius of curvature and located close to the junction edge (43) and may initiate the concave internal structure. This angle must be larger than 90° and smaller than 180° in order to promote a substantially complete emptying of the container. The radius of curvature of this angle is meant to compensate misalignment/assembly tolerances of the rigid part of the container (20) and the flexible sheet (28).The second angle α may be rounded with a strictly non-null radius of curvature located close to a bottom of the container and may initiate a substantial bottom plate of the first container. This substantial bottom plate may comprise at least one of the outlet, the channel and the filter. The radius of curvature of the second angle α is meant to promote a substantial emptying of the container by allowing the flexible sheet (28) to come into contact with the concave internal structure (24) smoothly, without creases. At least one of the first angle β' and the second angle α' (preferentially both) of the flexible sheet and their respective radius of curvature may be substantially similar to α and β (preferentially equal), in such a manner that the internal wall of the flexible sheet may match as much as possible the internal wall of the concave internal structure.

Furthermore, as described in the figure 7b, the concave internal structure (24) may comprise a first angle β and a second angle α. The first angle β may have a radius of curvature substantially null and located close to the junction edge (43) and may initiate the concave internal structure. This angle must be larger than 90° and smaller than 180° in order to promote a substantially complete emptying of the container. The second angle α may be rounded with a strictly positive radius of curvature located close to a bottom of the container and may initiate a substantial bottom plate of the first container. This substantial bottom plate may comprise at least one of the outlet, the channel and the filter. The radius of curvature of the second angle α is meant to promote a substantial emptying of the container by allowing the flexible sheet (28) to come into contact with the concave internal structure (24) smoothly, without creases. At least one of the first angle β' and the second angle α' (preferentially both) of the flexible sheet and their respective radius of curvature may be substantially similar to α and β (preferentially equal), in such a manner that the internal wall of the flexible sheet may match as much as possible the internal wall of the concave internal structure.

Furthermore, the flexible sheet may be thermoformed so as to have a substantial similar shape of the internal wall of the concave internal structure. The first container may further comprise a slope (P).

Thus, the shape and the characteristic of the concave internal structure and the flexible sheet may be configured to allow a substantial draining of the first storage compartment.

The second container (12) may comprise similar features to the first container (11).

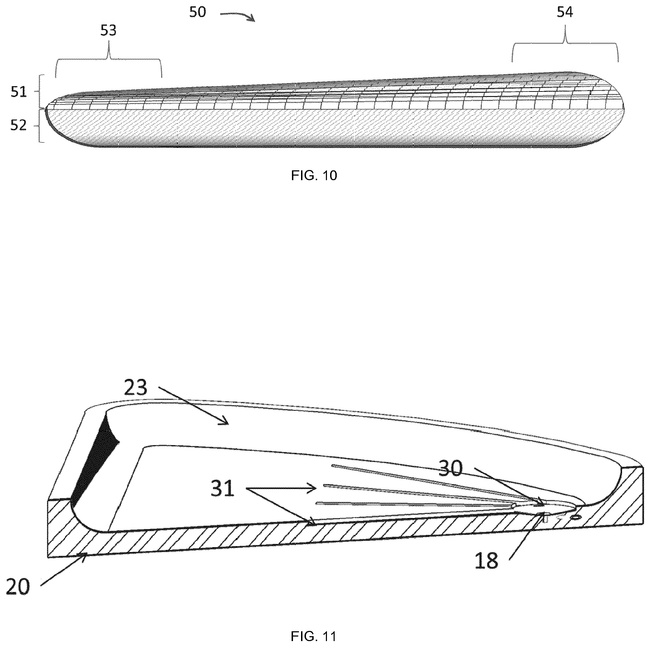

As described by the figure 11, the rigid part (20) of the first container may comprise one or more channel (31), and/or a cavity (30) at the outlet location (32). The cavity (30) and/or the channel (31) may be arranged in the internal wall of the rigid part (20) and may be configured to prevent a clogging of the outlet by the flexible sheet before the end of the delivery.

In order to prevent the clogging of the outlet (before the end of the delivery), at least one of the first and the second containers may be configured to enable a collapse of the flexible membrane in a determined manner, in a determined direction and/or in determined sequence. For example, the flexible membrane may collapse in a first instance to a remote location from the outlet and then the flexible sheet may collapse toward the outlet. As described by the figure 9a and 9b, the determined direction may be parallel to the length of the container.

Other examples of features which should prevent the clogging of the outlet (before the end of the delivery),:
- the width of the rigid part (20) close to the outlet location may be narrower than the farthest part or parts from the outlet,
- the outlet location may be arranged close to a vertical wall / internal edge of the container,
- the outlet location may not be arranged at or close to the centre of the container,
- the outlet location may be arranged as far as possible from the centre of the container,
- the flexible membrane may be more flexible close to the first end,
- the flexible membrane may be less flexible at the outlet location,
- the flexible membrane may comprise a determined shape at the outlet location (for example an additional cavity),
- the thermoforming of the flexible membrane may be done such as to exhibit a larger compliance far from the outlet and the smallest compliance around the outlet, and/or
- the thermoforming may be configured to limit the collapse at the outlet location.

The figure 10 shows an example of a container (50) having a flexible wall (51) and an opposite wall (52). The opposite wall defines an internal cavity in which the flexible wall is configured to come into contact. The flexible wall may be thermoformed. The opposite wall may be flexible or rigid. The first part (53) of flexible wall is thermoformed in such a manner to come in contact with a predetermined strain which is greater than the required strain for the second part (54) of the flexible wall. In other terms, the first part (53) of the flexible wall requires a plastic or elastic deformation of the flexible wall which is greater than the second part (54).

The material of the flexible membrane (single flexible membrane, first flexible membrane or second flexible membrane) may, for example, consist of one or more polymers of the following families: Polypropylene (PP), Polyethylene (PE), Ethylene Vinyl Alcohol (EVOH), Polyamide (PA), Polychlorotrifluoroethylene (PCTFE), Cyclic Olefin Copolymer (COC), Cyclic Olefin Copolymer elastomer (COC elastomer), Polycarbonate (PC), Ethylene Vynil Acetate (EVA), Polyvinyl Chloride (PVC), Polyvinylidene Chloride (PVDC), Polystyrene (PS), Polyethylene Terephthalate (PET), Thermoplastic Elastomer (TPE), Polymethacrylate (PMMA / MABS / MBS), Nitrile Butadiene Rubber (NBR), Natural Rubber (NR), Silicone, or other polymers. The flexible membrane may be thermoformed and may be manufactured for example by extrusion, blown film extrusion, coextrusion or lamination.

The rigid wall part may be an injection molded part. It may for example, consist of one or more polymers of the following families: Polypropylene (PP), Cyclic Olefin Copolymer (COC), Polymethacrylate (PMMA / MABS / MBS), Polyoxymethylene (POM), Acrylonitrile butadiene styrene (ABS), Copolyester (PCTG), Polyethylene Terephthalate (PET) or Polycarbonate (PC). Fillers can be used to reinforce the shell, e.g. glass fibers, Kevlar...

In order to reduce the cost of the rigid part of the container, the delivery system may comprise a first layer and a second layer. The first layer may comprise a first surface intended to be in contact with the solution and a second surface intended to be in contact with the second layer. Preferentially the first surface is arranged on another face (for example opposite face) than the face comprising the second surface. The second layer is intended to provide a determined mechanic property (for example a better rigidity) to the container. The first layer may be independent of (it may be removably coupled to) or secured to the second layer (for example rigidly fixed). In this case, the first layer may for example, consist of one or more polymers of the following families: Polypropylene (PP), Cyclic Olefin Copolymer (COC), Polymethacrylate (PMMA / MABS / MBS), Copolyester (PCTG), Polyethylene Terephthalate (PET) or Polycarbonate (PC). And the second layer may for example, consist of one or more polymers of the following families: Polyethelene (PE, HDPE, LDPE), Polypropylene (PP), Polyethylene Terephthalate (PETE or PET), Acrylonitrile-Butadiene-Styrene (ABS), Polyvinyl Chloride (PVC), Polyamide (PA), Acrylic (PMMA), Polylactic Acid (PLA), Polycarbonate (PC), Polystyrene (PS, GGPS, HIPS), Acetal, Polyoxymethylene (POM), Polyurethane (PU) or glass fibres...

The first container (11) may comprise a rigid part and a flexible part as described by the US patent application US20140166528, the content of the patent application is incorporated by reference in the present document.

The second and the third container may be configured to store a compressible fluid (for example in a compressed state) such as a propellant, for example consisting of one or more material of the following families: butane, propane, CFC11, CFC12, CFC114, HFA 134a, HFA 152a, HFA 227, Isobutene, n-butane, HFO1234yf, or HFO1234ze. Thus the flexible membrane (single flexible membrane, first flexible membrane or second flexible membrane) may be configured to be in contact with a compressible fluid such as listed above. In this case, the material (which may be intended to be in contact with the compressed fluid (propellant)), for example the second flexible membrane, may consist of one or more material of the following families: Natural Rubber, Styrene Butadiene, Ethylene Propylene (EPDM), Nitrile, Neoprene, Flurocarbon, Fluoro Silione, Urethane, SBR, Silicone, Butyl, Polyacrylate, Hypalon, Viton, Polyurethane, Fluorosilicone, Aflas, or Kalrez.

When the compressed fluid enters into the second storage compartment, the expansion of the compressed fluid induces shocks which may damage the flexible membrane of the first container or the solution. Thus, the second flexible membrane may be configured to insulate/protect the first flexible membrane and/or the solution from the thermal shock and/or expansion shock when the compressed fluid enters into the second storage compartment. An additional layer arranged between the first and the second flexible membrane may comprise a thermal insulating material.

At least one of the fluid compressed, the fluid pathway (5), the third container (13) and the second container (12) may be adapted to one or several features (volume, viscosity, active agent, life cycle, live time of the solution...) of the solution stored in the first container in order to infuse the solution during a predetermined time period.

The second container may further comprise an outlet configured to drain the second storage compartment at the end of use or delivery. This outlet may be closed by a valve device during the delivery and when the first storage compartment has been drained, this valve device may be open (automatically or by a user).

The first, the second and/or the third container may be arranged into the housing. In one embodiment, the rigid part (or at least a part) of one of the containers may be a part of the housing of the delivery system. In other terms, a part of the rigid part of one of the containers may be configured to be in contact with the external environment of the delivery system. Thus, the mechanical property of the rigid part of the container is designed in such a manner to provide a mechanical protection to the delivery system.

The housing may for example, consist of one or more polymers of the following families: Cyclic Olefin Copolymer (COC), Polymethacrylate (PMMA / MABS / MBS), Copolyester (PCTG), Polyethelene (PE, HDPE, LDPE), Polypropelene (PP), Polyethylene Terephthalate (PETE or PET), Acrylonitrile-Butadiene-Styrene (ABS), Polyvinyl Chloride (PVC), Polyamide (PA), Acrylic (PMMA), Polylactic Acid (PLA) Polycarbonate (PC), Polystyrene (PS, GGPS, HIPS), Acetal, Polyoxymethylene (POM), Polyurethane (PU) or glass fibers...

The rigid housing and/or at least a part of the container may be translucent or transparent and/or the rigid housing may comprise a window so as to see the container.

The rigid part of at least one of the first container (11) and the second container (12) may define a part of the housing (10) (which may be into contact with the external environment). At least a part of the rigid wall of the third container may be a part of the housing (10) (which may be into contact with the external environment).

Preferentially, the first container and the second container comprise symmetric forms at least in one dimension (for example length and/or width) in such a manner that the second flexible membrane substantially (preferentially perfectly) matches with the concave internal structure of the first container.

Preferentially, the first (resp. second) container or the housing/support of the first (resp. second) container may comprise edge and/or coupling devices (clip or screw or other fixing means) configured to rigidly fix (or to glue or to weld) to the second (resp. first) container or the housing/support of the second (resp. first) container and/or to the third container or the housing/support of the third container.

### 1.6 CONTAINERS ARRANGEMENT

Preferentially the first container is adjacent to the second container, more preferentially, the first movable wall is opposite to the second movable wall in such a manner that the first and the second movable walls may be in contact and in pressure communication.

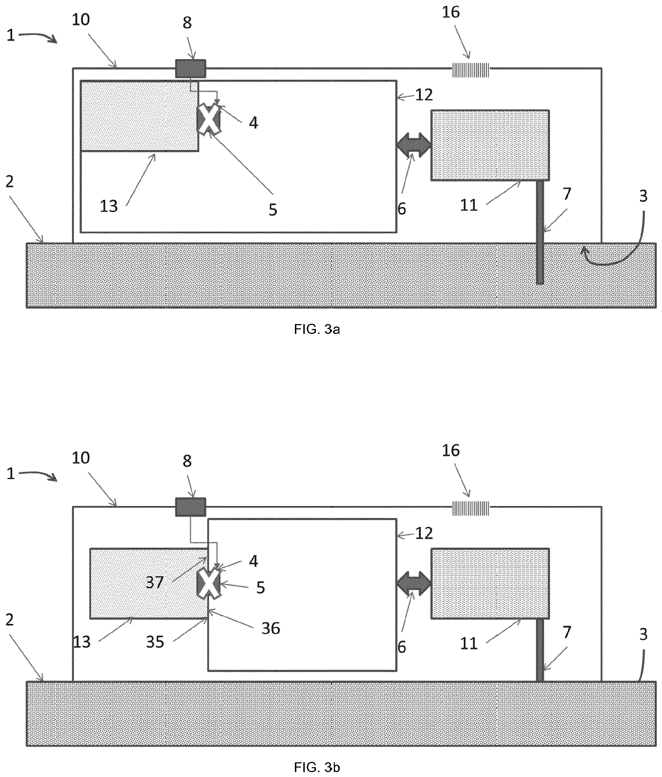

As disclosed by the figure 3a, the third container (13) may be arranged at least partially into the second storage compartment. As disclosed by the figure 3b, at least a part of the rigid wall (35) of the third container (13) may be at least a part of the rigid wall of the second container (12). In other terms, a first surface (36) of a rigid wall (35) defines a part of the second storage compartment and a second surface (37) of the same rigid wall (35) defines a part of the third storage compartment. The first surface (36) may be opposite to the second surface (37).

In one embodiment, at least one rigid part of one of containers is used as a rigid structure of the delivery system. In this case, the other elements of the delivery system may be secured to this rigid part (for example other container, housing, indicator device, transcutaneous device, skin-adherable unit).

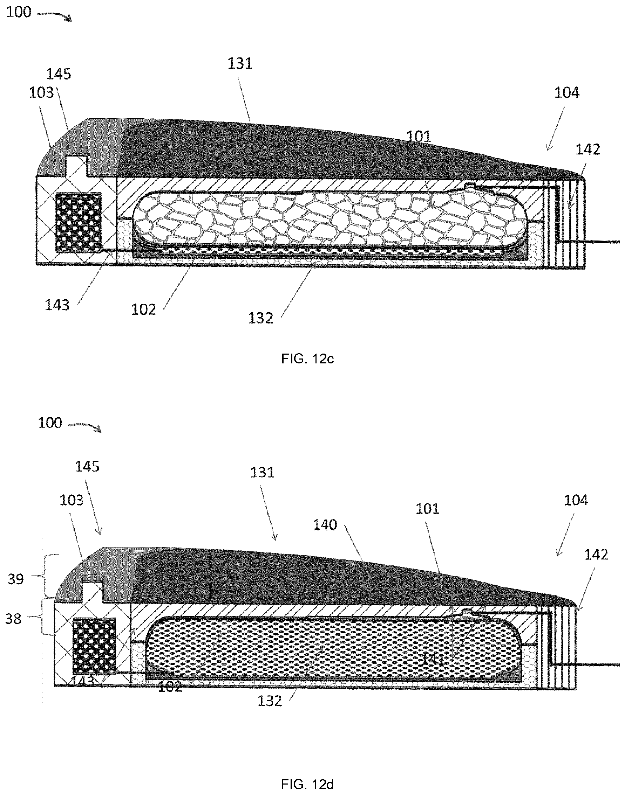

In one embodiment as described by the figures 12, the first container (101) and the second container (102) are arranged into a cavity defined by a rigid housing which may include at least one of a first rigid housing (131) and a second rigid housing (132). The rigid housing may comprise one or more vents (141) configured to insure pressure equilibrium between the cavity (140) and the external environment of the delivery system (100) when at least one of the first storage compartment volume and the second storage compartment volume changes. The first rigid housing (131) may comprise a part of a first fluid pathway (142) in which the solution flow or a passage in which said first fluid pathway is arranged. The second rigid housing (132) may comprise a part of a second fluid pathway (143) in which the compressed fluid flow or a passage in which said second fluid pathway is arranged.

The first rigid housing may comprise a concave internal structure configured to receive at least a part of the first container (101). The first container (101) may be secured to the first rigid housing (131). At least a part of the concave internal structure of the first rigid housing may be formed so as to match with a part of the external surface of the first container.

The third container (103) (or the housing comprising the third storage compartment) and/or the indicator device (104) may be secured to the rigid housing (131, 132).

The first container may be a first flexible pouch configured to be in fluid communication with the transcutaneous device and the second container may be a second flexible pouch configured to be in fluid communication with the third container. In this case, the edge of the flexible pouch may be rigidly fixed to the housing at least at two separate locations.

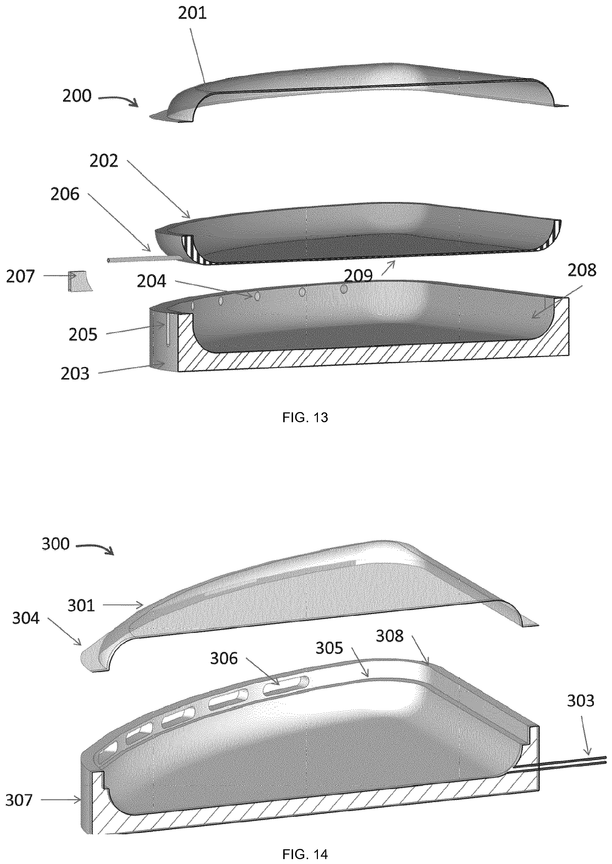

The figure 13 shows an exploded view of a container (the first or the second container) and a part of the housing. The container (200) comprises a flexible membrane (201) sealed on a rigid part (202) and both define the storage compartment. The container further comprises a fluid pathway (206) in fluid connection with the storage compartment. The rigid housing (203) is configured to improve the mechanical property of the rigid part (202) of the container. The rigid housing (203) comprises a concave internal structure (208) fitted to at least a part of the external surface (209) of the container. The rigid housing may further comprise one or more vent (204) and a passage (205) for the fluid pathway (206). The passage may be at least partially covered by a lid (207) or also used as a vent.

The rigid housing and/or at least a part of the container may be translucent or transparent and/or the rigid housing may comprise a window so as to see the container.

The figure 14 shows an exploded view of a container (300) (the first or the second container). The container (300) comprises a flexible membrane (301) sealed on a rigid part (302) and both define the storage compartment. The rigid part may further comprise a protrusion (308) having one or more vent (306) and/or a support (305) configured to seal/fix the edge (304) of the flexible membrane. The support (305) may be a groove, a slot, a rim, an edge and/or a sill of the rigid part (302). The protrusion (308) may extend along a peripheral edge of the cavity (of the container interface) and/or toward an opposite container. The container further comprises a fluid pathway (303) in fluid connection with the storage compartment.

At least a part of the external surface (307) of the rigid part (302) may be a part of the housing.

At least a part of the external surface (307) of the rigid part (302) may be a rigid structure or a frame configured to secure/attach on it other elements of the delivery system such as another container, indicator device, transcutaneous device and/or skin-adherable unit.

According to the figures 2 to 4, the containers may be arranged side by side.

The delivery system may comprise proximal part and a distal part (to the patient skin). Preferentially, the proximal part comprises a lower face and may be configured (for example at least a part of the lower face) to be coupled to the skin-adherable unit. The distal part comprises upper face and at least a part of the distal part (for example at least a part of the upper face) is configured to be visible (by the patient or other persons) when the delivery system is fixed on the patient skin.

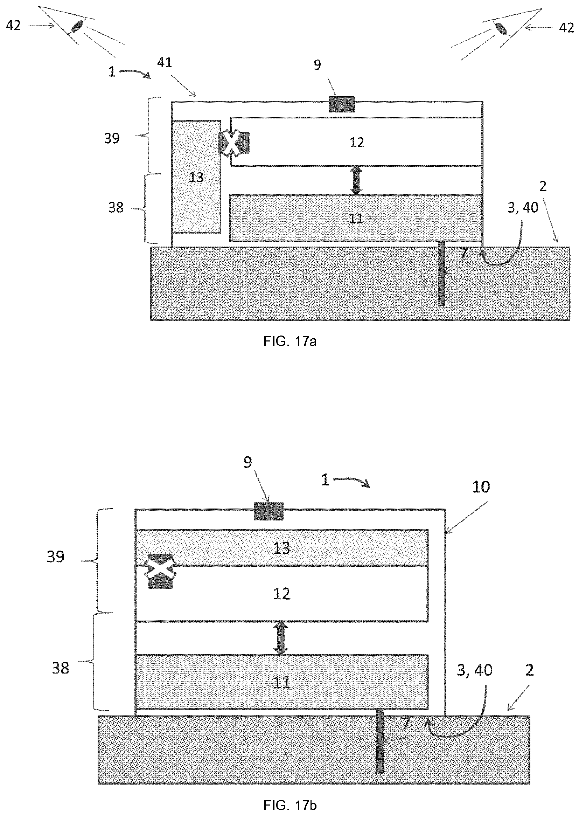

According to the figures 17a and 17b, the first container (11) is arranged at least partially in the proximal part (38) in order to simplify the fluid pathway connected to the injection device (7) and the second container (12) is arranged above the first container (11) for example at least partially in the distal part (39) of the delivery system (1). In this case, the third container (13) may be arranged above the second container (12) (as shown by the fig. 17b) or on the side of at least one of the first container and the second container (as shown by the fig. 17a). At least a part of the first container (for example a rigid part of the first container) forms/defines the lower face of the delivery system and may be secured or coupled to the skin-adherable unit. At least a part of the second and/or third container (preferentially a rigid part) may form/define the upper face of the delivery system. Preferentially, at least a part of the proximal part may comprise a window or a translucent or transparent material so to see the solution stored into the first container.

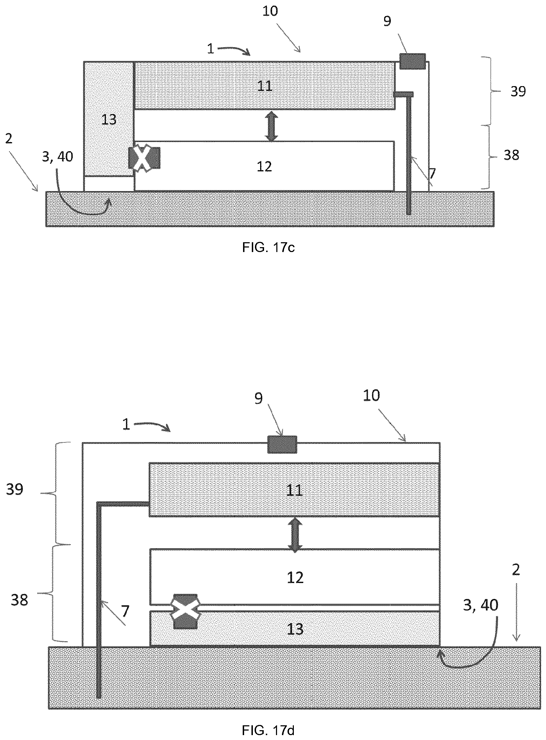

According to the figures 17c and 17d, the second container (12) is arranged at least partially in the proximal part (38) and the first container (11) is arranged above the second container (12) for example at least partially in the distal part (39) of the delivery system (1). In this case, the third container (13) may be arranged below the second container (12) (as shown by the fig. 17d) or on the side of at least one of the first container and the second container (as shown by the fig. 17c). At least a part of the first container (for example a rigid part of the first container) may form/define the upper face of the delivery system. At least a part of the second and/or third container (preferentially a rigid part) forms/defines the lower face of the delivery system and may be secured or coupled to the skin-adherable unit. Preferentially, at least a part of the distal part may comprise a window or a translucent or transparent material so as to see the solution stored into the first container, even if the delivery system is secured on the patient skin.

The indicator device is preferentially arranged in such a manner to be visible to the patient when the delivery system is secured on the patient skin.

### 1.7 EXAMPLES OF EMBODIMENTS

According to the figures 12a and 12b, the delivery system (100) comprises a first container (101), a second container (102) and a third container (103). The first container (101) comprises a first storage compartment (111) intended to store a solution. The second container (102) comprises a second storage compartment (112) intended to receive a propellant when the delivery system (100) is in delivery state. The first container (101) comprises a first flexible membrane (121) and the second container (102) comprises a second flexible membrane (122). The first flexible membrane (121) is arranged opposite the second flexible membrane (122). The first container and the second container are configured to be in pressure communication when the first flexible membrane and the second flexible membrane are in contact. The pressure transmission may be partial or total and may depend on elasticity of at least one of the first and the second flexible membranes. In the figure 12a, the first storage compartment (111) and the second storage compartment (112) are substantially empty.

The first storage compartment (111) is configured to be in fluid communication with a patient via a first fluid pathway (142) and optionally via a transcutaneous device (not shown). The second storage compartment (112) is configured to be in fluid communication with the third container (103) via the second fluid pathway (143).

The delivery system (100) may further comprise an indicator device (104) which may be in fluid or pressure communication with the first storage compartment (111). The delivery system (100) may further comprise a trigger device (145) configured to launch/initiate a delivery sequence. The delivery sequence may comprise a delivery step and an optional countdown step (before the delivery).

At least one of the first flexible membrane (121) and the second flexible membrane (122) defines the cavity of the containers' interface. The cavity (140) is vented by at least one vent (141).

The first container (101) may be arranged at least partially in the distal part (39) of the delivery system (100) and the second container (102) may be arranged at least partially in the proximal part (38) of the delivery system (100).

The figure 12c shows the same delivery system (100) but with a first container (101) filled with the solution. And the figure 12d shows the delivery system (100) with a first container (101) drained and a second container (102) filled with the compressed fluid.

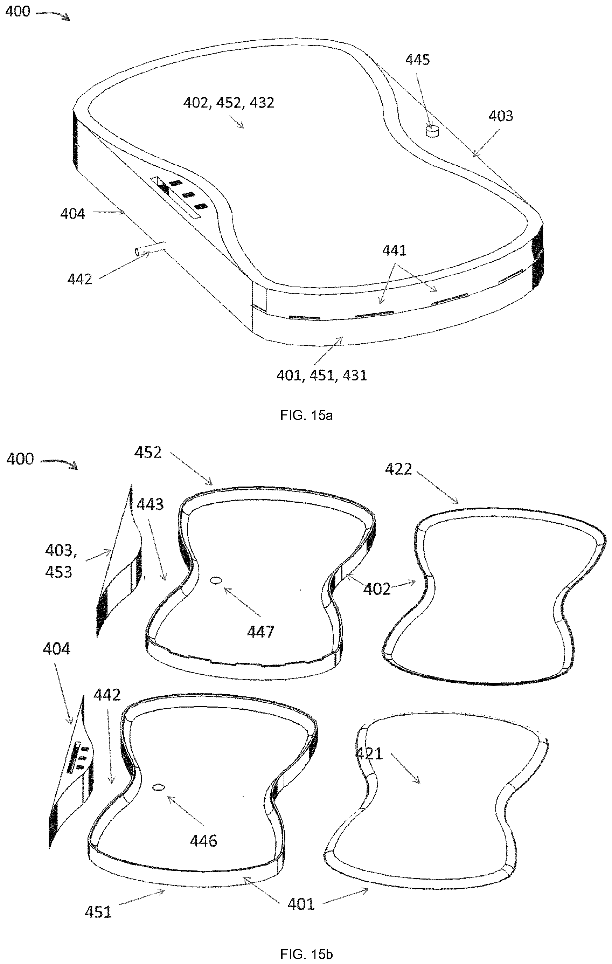

The figures 15a and 15b show another embodiment where at least one of the first container (401) and the second container (402) comprises a butterfly shape. The first container (401) and the second container (402) comprise symmetric forms at least in one dimension (for example length and/or width) in such a manner that the second flexible membrane (422) substantially (preferentially perfectly) matches with the internal wall of the rigid part (451) of the first container (401). The inlet (447) of second container is not arranged above/below the outlet (446) of the first container.

The delivery system comprises a first container (401) and a second container (402), both arranged opposite each other. The first container may comprise a rigid part (451) and a part of the rigid part (451) may act as a first rigid shell (431) of the delivery system (400). The second container may comprise a rigid part (452) and a part of the rigid part (452) may act as a second rigid shell (432) of the delivery system (400).

The delivery system may further comprise a third container (403) and a trigger device (445) arranged on the side of at least one of the first container (401) and the second container (402).

The delivery system may further comprise an indicator device (404) arranged on the side of at least one of the first container (401) and the second container (402).

The first rigid shell (431) may comprise a surface configured to be coupled or to be secured to a skin adherable unit (not shown). The indicator device may comprise visible information (LED, Message, level indicator) arranged on a visible part of the delivery system (400) when the delivery system is secured on the patient skin.

At least a part of the second rigid shell (432) may comprise an upper face of the delivery system (400).

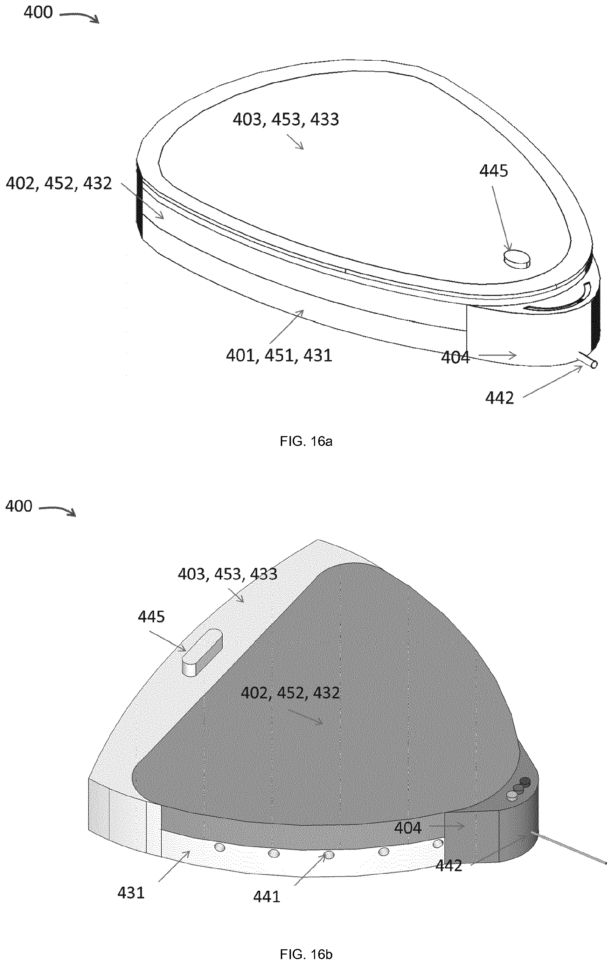

The figures 16a and 16b show a delivery system (400) having a substantial egg shape. In the embodiment of the figure 16a, the containers are stacked on top of each other. In the embodiment of the figure 16b, at least one of the first container (401) and the second container (402) comprises one or more vent (441). The first container may be arranged at least partially into a first rigid shell (431). The second container (402) may comprise a rigid part (452) having a surface configured to form or define at least a part of the rigid shell (432).

## Claims

1. A delivery system (1) configured to be secured on a patient skin comprising:
- a first container (11) having a first movable wall (6a) and a first storage compartment (11a) intended to store a medical fluid,
- a second container (12) having a second movable wall (6b) and a second storage compartment (12a), and
- a skin adherable unit (3) configured to secure the first container (11) and the second container (12) to the patient skin,
wherein the first movable wall (6a) and the second movable wall (6b) are facing each other and define a containers' interface (6) having a cavity (44);
wherein the system (1) further comprises a vent device (16) configured to allow a pressure equilibration between the cavity (44) of the containers' interface and the outside of the delivery system when at least one of the first movable wall (6a) and the second movable wall (6b) moves.

2. System according to the claim 1, wherein at least one of the first container (11) and the second container (12) comprises a rigid part (20, 25).

3. System according to the previous claim, wherein the rigid part (20, 25) extends along at least one length of at least one of the first container (11) and the second container (12) in at least one dimension.

4. System according to any one of the previous claims, wherein at least one of the first container (11) and the second container (12) comprises a protrusion (308) in which is arranged the vent device (16, 306).

5. System according to the claim 4, wherein the protrusion (308) extends toward the opposite container

6. System according to the claim 4, wherein the protrusion (308) extends along at least a part of the peripheral edge of the cavity.

7. System according to any one of the previous claims, wherein at least one of the first container (11) and the second container (12) comprises a concave internal structure (23, 24) in which at least one of the first movable wall (6a) and the second movable wall (6b) is configured to move.

8. System according to the previous claim, wherein the concave internal structure (23, 24) is a rigid structure and comprises a rigid internal wall of the storage compartment.

9. System according to any one of the previous claims, wherein the first container (11) and the second container (12) are in pressure communication only when the first movable wall (6a) and the second movable wall (6b) are at least partially in contact.

10. System according to any one of the previous claims, wherein the cavity (44) of the containers' interface comprises a variable volume depending on the movement of at least one of the first movable wall (6a) and the second movable wall (6b).

11. System according to any one of the previous claims further comprising a third container (13) configured to store a compressed fluid in a third storage compartment.

12. System according to the previous claim further comprising a fluid pathway (5) and a fluid communication device (4) configured to allow a fluid communication between the third storage compartment (13a) and the second storage compartment (12a) when the fluid communication device is in open status.

13. System according to the previous claim further comprising a trigger device (8) operable coupled with the fluid communication device and configured to change the status of the fluid communication device.

14. System according to the previous claim further comprising a countdown mechanism configured to activate the trigger device (8) after a predetermined time period.

15. System according to any one of the previous claims further comprising a housing (203) including a concave internal structure (208) fitted to at least a part of the external surface of at least one of the first container (11, 200) and the second container (12, 200).
